# EUROPÄISCHE PATENTSCHRIFT

(11) **EP 1 321 432 B1**
(45) Veröffentlichungstag und Bekanntmachung des Hinweises auf die Patenterteilung: **08.03.2006**
(21) Anmeldenummer: 02024335.8
(22) Anmeldetag: 02.11.2002
(51) Int. Cl.: C01G 23/00, C01G 23/07, C09C 1/36, A61Q 17/04, A61K 8/25, A61K 8/29

(54) **Flammenhydrolytisch hergestelltes Silicium-Titan-Mischoxidpulver mit an der Oberfläche angereichertem Siliciumdioxid, dessen Herstellung und Verwendung**
Silicon-titanium mixed-oxide prepared by flame hydrolysis and having a surface enriched in silicon dioxide, its production and use thereof
Oxyde mixte silicium-titane préparé par hydrolyse à la flamme et dont la surface est enrichie de dioxyde de silicium, sa fabrication et utilisation

(30) Priorität: 22.12.2001 DE 10163938
(43) Veröffentlichungstag der Anmeldung: 25.06.2003
(73) Patentinhaber: Degussa AG, 40474 Düsseldorf (DE)
(72) Erfinder: Mörters, Martin, Dr., 63450 Hanau (DE); Hemme, Ina, Dr., 63450 Hanau (DE); Hasenzahl, Steffen, Dr., 63454 Hanau (DE); Diener, Uwe, 63538 Grosskrotzenburg (DE); Habermann, Herbert, 63599 Biebergemünd (DE)

(56) Entgegenhaltungen:
- DE-A- 4 235 996
- LIU A T ET AL: "PRODUCTION OF FUMED OXIDES BY FLAME HYDROLYSIS" 1986 , NOVEL CERAMIC FABRICATION PROCESSES AND APPLICATIONS.;CAMBRIDGE, ENGL , BRITISH CERAMIC PROCEEDINGS 1986 INST OF CERAMICS, STOKE-ON-TRENT, ENGL, PAGE(S) 1 - 10 XP001179377 3.1 und 4.2.3
- CHENG-HUNG HUNG ET AL: "Formation of mixed oxide powders in flames. I. TiO/sub 2/-SiO/sub 2/" JOURNAL OF MATERIALS RESEARCH, JULY 1992, USA, Bd. 7, Nr. 7, Seiten 1861-1869, XP009025974 ISSN: 0884-2914

## Beschreibung

Gegenstand der Erfindung ist flammenhydrolytisch hergestelltes Silicium-Titan-Mischoxidpulver, welches an der Oberfläche mit Siliciumdioxid angereichert ist, dessen Herstellung und Verwendung.

Titandioxid findet weite Verbreitung in Sonnenschutzmitteln. Seine Wirkung beruht im wesentlichen auf Reflexion, Streuung und Absorption der schädigenden UV-Strahlung und hängt wesentlich von der Primärpartikelgröße der Metalloxide ab.

Nachteilig hierbei ist seine photokatalytische Aktivität, durch die Reaktionen ausgelöst werden, die zu Veränderungen von Bestandteilen eines Sonnenschutzmittels führen können.

Es wird versucht die photokatalytische Aktivität zu verringern ohne die UV-abschirmenden Eigenschaften zu reduzieren, indem man Titandioxid zum Beispiel mit einer Hülle aus Siliciumdioxid umgibt.

Nachteilig ist, dass für die Herstellung solcher umhüllter Partikel zwei Reaktionsschritte nötig sind, mit entsprechend hohem technischen Aufwand. Die zwei Schritte umfassen die Herstellung der Titandioxidpartikel und die Ausbildung der Hülle, in der Regel in einem wässerigen EP-A-1284277) oder wässerigalkoholischen (EP-A-0 988 853) Medium durch Hydrolyse eines Siliciumdioxidvorläufers.

In DE-A-4235996 wird ein flammenhydrolytisch hergestelltes Silicium-Titan-Mischoxid und dessen Verwendung als UV-Absorber in Sonnenschutzmitteln beschrieben. Dieses Pulver kann zwar in einem einzigen Reaktionsschritt hergestellt werden, die photokatalytische Aktiviät, bezogen auf flammenhydrolytisch hergestelltes Titandioxid, wird jedoch weniger reduziert als bei mit Siliciumdioxid umhülltem Titandioxid.

Die Aufgabe der Erfindung ist es, ein Pulver bereitzustellen, das die Nachteile des Standes der Technik vermeidet. Insbesonders soll es eine niedrige photokalytische Aktivität aufweisen und einfach herzustellen sein.

Gegenstand der Erfindung ist ein flammenhydrolytisch hergestelltes Silicium-Titan-Mischoxidpulver, welches dadurch gekennzeichnet ist, dass das Gewichtsverhältnis von Siliciumdioxid / Titandioxid auf der Oberfläche der Primärpartikel größer ist als im Gesamtprimärpartikel.

Das Gewichtsverhältnis von Siliciumdioxid/Titandioxid auf der Oberfläche kann zum Beispiel durch Röntgen-induzierte Photoelektronen-Spektroskopie (XPS-Analyse) des Pulvers bestimmt werden. Zusätzliche Information über die Oberflächenzusammensetzung kann durch energiedispersive Röntgenstrahlung (TEM-EDX-Analyse)von einzelnen Primärpartikeln bestimmt werden.

Das Gewichtsverhältnis im Gesamtprimärpartikel wird durch chemische oder physikalisch-chemische Methoden, z.B. Röntgenfloureszenzanalyse, des Pulvers bestimmt.

Der Gesamtprimärpartikel schliesst den Anteil an Siliciumdioxid und Titandioxid an der Oberfläche mit ein. Es ist der Partikel der im flammenhydrolytischen Prozess zunächst gebildet wird. Die Primärpartikel können im weiteren Reaktionsverlauf zu kettenartigen Aggregaten und diese weiter zu Agglomeraten zusammenwachsen. Je nach Wahl der Reaktionsbedingungen können bei der Synthese auch im wesentlichen sphärische Partikel erhalten werden.

Unter Mischoxid ist die innige Vermischung von Titandioxid und Siliciumdioxid auf atomarer Ebene unter Bildung von Si-O-Ti-Bindungen zu verstehen. Daneben können die Primärpartikel auch Bereiche von Siliciumdioxid neben Titandioxid aufweisen.

Das erfindungsgemäße Silicium-Titan-Mischoxidpulver kann ferner Spuren von Verunreinigungen aus den Ausgangsstoffen wie auch durch den Prozess hervorgerufen Verunreinigungen aufweisen. Diese Verunreinigungen können bis zu 0,5 Gew.%, in der Regel jedoch nicht mehr als 100 ppm, betragen.

Das Gewichtsverhältnis von SiO₂/TiO₂, bezogen auf den Gesamtprimärpartikel, kann im erfindungsgemäßen Pulver von 0,01 bis 99 betragen. In diesem Bereich ist das Gewichtsverhältnis von Siliciumdioxid / Titandioxid auf der Oberfläche der Primärpartikel größer als im Gesamtprimärpartikel.

Unter der Maßgabe, dass das Gewichtsverhältnis von SiO₂/TiO₂ zwischen 0,05 und 4 liegt, bezogen auf den Gesamtprimärpartikel, kann in einer besonderen Ausführungsform das Gewichtsverhältnis von SiO₂/TiO₂ auf der Oberfläche des Primärpartikels einen Wert annehmen, der sich aus der Formel

**[SiO**_{**2**}**/TiO**_{**2**}**]** _{**Oberfläche**} = **9,3[SiO**_{**2**}**/TiO**_{**2**}**]**_{**Gesamtprimärpartikel**} ^{**1,24**}

berechnen läßt, wobei die absolute Abweichung des Gewichtsverhältnisses [SiO₂/TiO₂] _{Oberfläche} von der angegebenen Formel maximal 1,5 beträgt.

So entspricht ein SiO₂/TiO₂-Verhältnis von 0,1 im Gesamtprimärpartikel einem Oberflächenverhältnis von 0,64. Der experimentell ermittelte Wert liegt bei 0,57, die absolute Abweichung demnach 0,07 (siehe Beispiel 2).

Das erfindungsgemäße Pulver kann BET-Oberflächen zwischen 10 und 300 m²/g aufweisen. Die BET-Oberfläche kann in einem bestimmten Bereich durch die Prozessparameter variiert werden. Darüber hinaus hat das Gewichtsverhältnis SiO₂/TiO₂ einen Einfluß auf die BET-Oberfläche. Eine höhere BET-Oberfläche wird, bei nicht veränderten Prozessparametern, mit steigendem SiO₂/TiO₂-Verhältnis leichter zu realisieren sein.

Ein weiterer Gegenstand der Erfindung ist ein Verfahren zur Herstellung des erfindungsgemäßen Silicium-Titan-Mischoxidpulvers. Dabei führt man einen Strom aus einem dampfförmigen Titandioxidvorläufer und Sauerstoff oder einem Sauerstoff enthaltendem Gas und Wasserstoff, und einen zweiten Strom aus einem dampfförmigen Siliciumdioxidvorläufer und einem Trägergases aus Sauerstoff, einem Sauerstoff enthaltenden Gas und/oder einem Inertgases, getrennt in den Reaktionsraum eines Brenners, wie er zu Herstellung pyrogener Oxide bekannt ist, wo das Gemisch verbrennt, danach das feste Mischoxidpulver und heißen Gase abkühlt und die Gase vom Feststoff abtrennt.

Das Gemisch aus dem Siliciumdioxidvorläufer und dem Trägergas kann an einer oder mehreren Stellen im Reaktionsraum zugeführt werden. Das Mischoxidpulver kann gegebenenfalls durch eine Wärmebehandlung mittels Wasserdampf angefeuchteter Gase gereinigt werden.

Als Titandioxidvorläufer und Siliciumdioxidvorläufer können anorganische und/oder organische Verbindungen eingesetzt werden. Als anorganische Verbindungen eignen sich besonders Halogenide. Als organische Verbindungen eignen sich besonders Siloxane wie Hexamethyldisiloxan. Besonders geeignet sind Siliciumtetrachlorid und Titantetrachlorid.

In einer besonderen Ausführungsform kann als Titandioxidvorläufer Titantetrachlorid und als Siliciumdioxidvorläufer Siliciumtetrachlorid in einer Weise eingeleitet werden, dass sich das Gewichtsverhältnis SiO₂/TiO₂ auf der Oberfläche durch das Gewichtsverhältnis SiCl₄/TiCl₄ gemäß der Formel

**[SiO**_{**2**}**/TiO**_{**2**}**]**_{**Oberfläche**} = **7,3 [SiCl**_{**4**}**/TiCl**_{**4**}**]**^{**1,28**}**,**

mit einer absoluten Abweichung des Gewichtsverhältnisses [SiO₂/TiO₂] _{Oberfläche} von der angegebenen Formel von maximal 1,5 einstellt, mit der Maßgabe, dass das Gewichtsverhältnis SiO₂/TiO₂ im späteren Pulver zwischen 0,05 und 4, bezogen auf den Gesamtprimärpartikel, beträgt.

Ein weiterer Gegenstand der Erfindung sind Sonnenschutzmittel, die das erfindungsgemäße Mischoxidpulver in einem Anteil von 0,01 und 25 Gew.-% enthalten. Daneben kann das erfindungsgemäße Sonnenschutzmittel in Mischungen mit bekannten anorganischen UV-absorbierenden Pigmenten und/oder chemischen UV-Filtern eingesetzt werden.

Als bekannte UV-absorbierende Pigmente kommen Titandioxide, Zinkoxide, Aluminiumoxide, Eisenoxide, Siliciumdioxid, Silicate, Ceroxide, Zirkoniumoxide Bariumsulfat oder Gemische davon in Betracht.

Als chemische UV-Filter kommen alle dem Fachmann bekannten wasser- oder öllöslichen UVA- als auch UV-B-Filter in Frage, von denen exemplarisch, jedoch nicht limitierend Sulfonsäurederivate von Benzophenonen und Benzimidazolen Derivate des Dibenzoylmethans, Benzylidencampher und dessen Derivate, Derivate der Zimtsäure und deren Ester, oder Ester der Salizylsäure genannt seien.

Die erfindungsgemäßen Sonnenschutzmittel können ferner die dem Fachmann bekannten Lösungsmittel wie Wasser, ein- oder mehrwertige Alkohole, kosmetische Öle, Emulgatoren, Stabilisatoren, Konsistenzregler wie Carbomere, Cellulosederivate, Xanthan-Gum, Wachse, Bentone, pyrogene Kieselsäuren und weitere in Kosmetika übliche Stoffe wie Vitamine, Antioxidantien, Konservierungsstoffe, Farbstoffe und Parfums enthalten.

Typischerweise kann das erfindungsgemäße Sonnenschutzmittel als Emulsion (O/W, W/O oder multipel), wässeriges oder wässerig-alkoholisches Gel oder Ölgel vorliegen, und in Form von Lotionen, Cremes, Milchsprays, Mousse, als Stift oder in anderen gebräuchlichen Formen angeboten werden.

Der allgemeine Aufbau von Sonnenschutzmitteln ist darüber hinaus in A. Domsch, "Die kosmetischen Präparate", Verlag für chemische Industrie (Hrsg. H. Ziolkowsky), 4. Aufl., 1992 oder N.J. Lowe und N.A. Shaat, Sunscreens, Development, Evaluation and Regulatory Aspects, Marcel Dekker Inc., 1990 beschrieben.

Ein weiterer Gegenstand der Erfindung ist die Verwendung des Mischoxidpulvers gemäß den Ansprüchen 1 bis 4 als UV-Filter, zur Herstellung von Dispersionen, in Prozessen zum chemisch-mechanischen Polieren (CMP-Anwendung), in der Glasherstellung, als katalytisch aktive Substanz, als Katalysatorträger, Verwendung in Tonern und als Additiv in der Silikon- und Kautschukindustrie.

### Beispiele

### Beispiel 1:

Es werden 0,688 kg/h TiCl₄ in einem Verdampfer bei ca. 200°C verflüchtigt und der Chloriddampf mittels Stickstoff in die Mischkammer eines Brenners geleitet. Dort wird der Gasstrom mit 0,283 Nm³/h Wasserstoff und 0,837 Nm³/h getrockneter Luft (=Luftstrom 1) gemischt. In einem weiteren Verdampfer werden 0,232 kg/h SiCl₄ bei ca. 100 °C verdampft und mittels 0,763 Nm³/h trockener Luft (= Luftstrom 2) in den Brenner geführt.
Der Brenner besteht aus drei konzentrischen Rohren. Durch das innere Rohr (Innendurchmesser 4 mm) wird das Gemisch aus Luft und gasförmigen SiCl₄ der Flamme zugeführt. Durch das mittlere Rohr (Durchmesser 7 mm) wird das Gemisch aus TiCl₄, Luft und Wasserstoff aus der Mischkammer der Flamme zugeführt. Für beide Rohre ergeben sich bei einer Brennertemperatur von 230°C somit Austrittsgeschwindigkeiten von ca. 35,8 m/s. Durch das äußere Rohr wird als Mantelgas 0,05 Nm³/h Wasserstoff zugeführt.

Die Gase werden in der Reaktionskammer verbrannt und in einer anschließenden Koagulationsstrecke auf ca. 110°C abgekühlt. Das entstandene Mischoxid wird anschließend in einem Filter abgeschieden. Durch eine Behandlung des Pulvers mit feuchter Luft bei ca. 500-700°C wird anhaftendes Chlorid entfernt.

Das entstandene Oxidpulver weist ein Gewichtsverhältnis SiO₂/TiO₂ von 0,28 auf. Die BET-Oberfläche beträgt 72 m²/g. Hochauflösende TEM-Aufnahmen mit EDX-Analyse zeigen, daß die Oberfläche (Stelle A in Abbildung) ein Gewichtsverhältnis SiO₂/TiO₂ von 2 aufweist und von amorpher Natur ist. Die Dicke dieser Schicht beträgt ca. 4 nm.

Die TEM-Aufnahme des nach Beispiel 1 hergestellten Pulvers zeigt ferner einen kristallinen Kern (Stelle B in Abbildung). EDX-Analysen zeigen, daß dieser weitestgehend aus Titandioxid besteht. Durch Röntgenbeugung läßt sich ein Verhältnis der Titandioxidmodifikationen Anatas/Rutil von 80:20 ermitteln.

### Beispiele 2-10:

Weitere Versuche werden entsprechend der Beschreibung in Beispiel 1 durchgeführt. Variiert werden dabei das Verhältnis SiCl₄/TiCl₄ und die Gasmengen entsprechend Tabelle 1, wodurch sich die in Tabelle 2 zusammengefaßten Pulvereigenschaften ergeben.

**Tabelle 1: Anlageneinstellungen für die Beispiele 1-10**

| Beispiel | H₂ über Mischkammer | H₂ über Mantel | Luftstrom 1 | Luftstrom 2 | TiCl₄ | SiCl₄ | SiCl₄/ TiCl₄ | SiO₂/TiO₂ Gesamtpartikel |
|---|---|---|---|---|---|---|---|---|
| | Nm³/h | Nm³/h | Nm³/h | Nm³/h | kg/h | kg/h | | |
| 1 | 0,283 | 0,050 | 0,837 | 0,763 | 0,688 | 0,232 | 0,34 | 0,28 |
| 2 | 0,300 | 0,225 | 1,974 | 1,605 | 1,538 | 0,192 | 0,12 | 0,10 |
| 3 | 0,566 | 0,100 | 1,664 | 1,536 | 1,371 | 0,398 | 0,29 | 0,24 |
| 4 | 0,307 | 0,100 | 1,470 | 1,205 | 1,000 | 0,396 | 0,40 | 0,33 |
| 5 | 0,566 | 0,100 | 1,268 | 1,232 | 1,193 | 0,593 | 0,50 | 0,42 |
| 6 | 0,566 | 0,100 | 1,688 | 1,512 | 1,195 | 0,595 | 0,50 | 0,42 |
| 7 | 0,566 | 0,100 | 1,714 | 1,486 | 1,000 | 0,800 | 0,81 | 0,68 |
| 8 | 0,409 | 0,050 | 1,967 | 1,533 | 0,593 | 0,690 | 1,16 | 0,98 |
| 9 | 0,566 | 0,100 | 1,759 | 1,441 | 0,696 | 1,196 | 1,72 | 1,44 |
| 10 | 0,566 | 0,050 | 1,779 | 1,421 | 0,480 | ,320 | 2,75 | 2,31 |

Bei allen aufgeführten Experimenten wird durch TEM-EDX-Analyse oder XPS-Analyse eine deutliche Anreicherung von SiO₂ an der Oberfläche der Primärpartikel festgestellt.

**Tabelle 2: Pulvereigenschaften für die Beispiele 1-10:**

| Beispiel | BET-Oberfläche | SiO₂/TiO₂ Gesamtpartikel | SiO₂/TiO₂ Oberfläche exp.(*) | SiO₂/TiO₂ Oberfläche ber. | Abweichung exp./ber. absolut |
|---|---|---|---|---|---|
| | m²/g | | | | |
| 1 | 72 | 0,28 | 2,03 | 1,81 | 0,22 |
| 2 | 72 | 0, 10 | 0,64 | 0,51 | 0,13 |
| 3 | 78 | 0,24 | 1,63 | 1,49 | 0,14 |
| 4 | 77 | 0,33 | 2,13 | 2,23 | 0,10 |
| 5 | 65 | 0,42 | 2,85 | 2,98 | 0,13 |
| 6 | 94 | 0,42 | 3,00 | 2,98 | 0,02 |
| 7 | 86 | 0,68 | 5,25 | 5,55 | 0,30 |
| 8 | 157 | 0,98 | 10,11 | 8,83 | 1,28 |
| 9 | 99 | 1,44 | 15,67 | 14, 56 | 1,10 |
| 10 | 152 | 2,31 | 24,80 | 26,59 | 1,79 |

| | | | | | |
|---|---|---|---|---|---|
| * bestimmt durch XPS-Analyse | | | | | |

### Beispiel 11: Vergleichsbeispiel

Es werden 1,331 kg/h TiCl₄ in einem Verdampfer bei ca. 200°C verflüchtigt und 0,332 kg/h SiCl₄ in einem weiteren Verdampfer bei ca. 100°C verflüchtigt. Die Chloriddämpfe werden mittels Stickstoff in die Mischkammer eines Brenners bekannter Bauart geleitet. Dort wird der Gasstrom mit 0,182 Nm³/h Wasserstoff und 2,675 Nm³/h getrockneter Luft gemischt. Die Gase werden gemeinsam in die Reaktionskammer geleitet und verbrannt. Die Stützflamme (Mantel) wird dabei mit 0,225 Nm³/h Wasserstoff gespeist. In einer anschließenden Koagulationsstrecke werden die Reaktionsprodukte auf ca. 110°C abgekühlt. Das entstandene Mischoxid wird anschließend in einem Filter abgeschieden. Durch eine Behandlung des Pulvers mit feuchter Luft bei ca. 500-700°C wird anhaftendes Chlorid entfernt.

Das entstandene Oxidpulver enthält insgesamt 17,3 Gew.-% SiO₂ und 82,7 Gew.-% TiO₂. Die BET-Oberfläche beträgt 51 m²/g. TEM-EDX-Analysen zeigen keinen signifikanten Konzentrationsunterschied zwischen dem Inneren der Primärpartikel und der Oberfläche der Primärpartikel.

### Beispiel 12: Photokatalytische Aktivität

Bei der Bestimmung der photokatalytischen Aktivität wird die zu messende Probe in 2-Propanol suspendiert und 1 Stunde mit UV-Licht bestrahlt. Danach wird die Konzentration an gebildetem Aceton gemessen.

Ca. 250 mg (Genauigkeit 0,1 mg) der aus den Beispielen und Vergleichsbeispielen erhaltenen Partikel werden mit einem Ultra-Turrax-Rührer in 350 ml (275,1g) 2-Propanol suspendiert. Diese Suspension wird mittels einer Pumpe über einen auf 24°C temperierten Kühler in einen zuvor mit Sauerstoff gespülten Photoreaktor aus Glas mit einer Strahlungsquelle gefördert.

Als Strahlungsquelle dient z.B. eine Hg-Mitteldruck-Tauchlampe vom Typ TQ718 (Heraeus) mit einer Leistung von 500 Watt. Ein Schutzrohr aus Borsilikatglas begrenzt die emittierte Strahlung auf Wellenlängen >300nm. Die Strahlungsquelle ist außen von einem mit Wasser durchströmtem Kühlrohr umgeben.

Sauerstoff wird über einen Durchflußmesser in den Reaktor eindosiert. Mit dem Einschalten der Strahlungsquelle wird die Reaktion gestartet. Bei Reaktionsende wird sofort eine kleine Menge der Suspension entnommen, filtriert und mittels Gaschromatographie analysiert.

Beispiel 3 zeigt nach einer Stunde einen Acetongehalt von 131 ppm. Zum Vergleich wird das gleiche Experiment mit einer Dispersion von reinem, pyrogenem Titandioxid (Degussa P25, BET-Oberfläche 50 m²/g) durchgeführt. Im gleichen Zeitraum werden 2303 ppm Aceton gebildet.

Die photokatalytische Aktivität wird bei Verwendung des erfindungsgemäßen Pulvers also auf 5,7% der Aktivität von reinem Titandioxid reduziert.

### Beispiel 13: Sonnenschutzmittel

Mit der in Tabelle 3 gezeigten Rezeptur wurde ein Sonnenschutzmittel mit 4 Gew.-% des erfindungsgemäßen Pulvers nach Beispiel 3 hergestellt.

**Tabelle 3: Sonnenschutzmittel enthaltend das erfindungsgemäße Pulver aus Beispiel 3**

| Phase | Bestandteil | Gew.-% |
|---|---|---|
| A | Isolan GI 34 | 3,0 |
| | Rizinusöl | 1,2 |
| | Tegesoft OP | 10,0 |
| | Tegesoft Liquid | 5,0 |
| | Glycerin 86% | 3,0 |
| B | Paracera W80 | 1,8 |
| | Isohexadecan | 5,0 |
| C | Partikel nach Beispiel 3 | 4,0 |
| D | Magnesiumsulfat | 0,5 |
| | VE-Wasser | 66,5 |

Phase A wird in einem Mischer auf 70°C erwärmt. Nach dem Aufschmelzen auf einer Magnetheizplatte bei 80°C wird Phase B zu Phase A gegeben. Die Phase C wird mit ca. 300 U/min und unter Vakuum in die Ölphase eingerührt. Phase D wird ebenfalls auf 70°C erwärmt und unter Vakuum der Mischung aus A-C zugefügt.

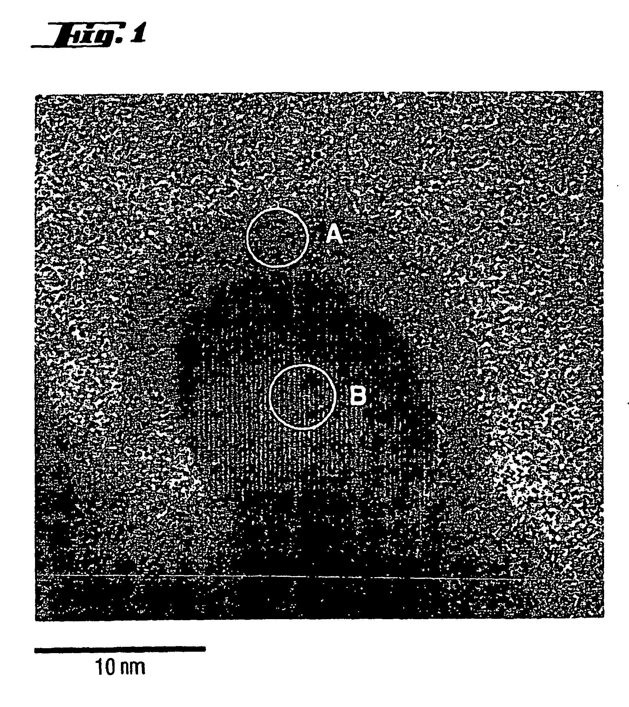

## Patentansprüche

1. Flammenhydrolytisch hergestelltes Silicium-Titan-Mischoxidpulver, **dadurch gekennzeichnet, dass** das Gewichtsverhältnis von Siliciumdioxid / Titandioxid auf der Oberfläche der Primärpartikel größer ist als im Gesamtprimärpartikel.

2. Pulver nach Anspruch 1, **dadurch gekennzeichnet, dass** das Gewichtsverhältnis von SiO₂/TiO₂ von 0,01 bis 99, bezogen auf den Gesamtprimärpartikel, ist.

3. Pulver nach Anspruch 1 oder 2, **dadurch gekennzeichnet, dass** bei einem Gewichtsverhältnis von SiO₂/TiO₂ 0,05 bis 4, bezogen auf den Gesamtprimärpartikel, das Gewichtsverhältnis SiO₂/TiO₂ auf der Oberfläche den Wert gemäß der Formel
[SiO₂/TiO₂] _{Oberfläche} = 9,3 [SiO₂/TiO₂] _{Gesamtprimärpartikel}^{1,24}
annimmt, wobei die absolute Abweichung des Gewichtsverhältnisses [SiO₂/TiO₂] _{Oberfläche} von der angegebenen Formel maximal 1,5 beträgt.

4. Pulver nach den Ansprüchen 1 bis 3, **dadurch gekennzeichnet, dass** deren BET-Oberfläche zwischen 10 und 300 m²/g liegt.

5. Verfahren zur Herstellung des Pulvers gemäß den Ansprüchen 1 bis 4, **dadurch gekennzeichnet, dass** man einen Strom aus einem dampfförmigen Titandioxidvorläufer und Sauerstoff oder einem Sauerstoff enthaltendem Gas und Wasserstoff, und einen zweiten Strom aus einem dampfförmigen Siliciumdioxidvorläufer und einem Trägergases aus Sauerstoff, einem Sauerstoff enthaltenden Gas und/oder einem Inertgases, getrennt in den Reaktionsraum eines Brenners, wie er zu Herstellung pyrogener Oxide bekannt ist, führt und dort verbrennt, danach das feste Mischoxidpulver und heißen Gase abkühlt und die Gase vom Feststoff abtrennt.

6. Verfahren nach Anspruch 5, **dadurch gekennzeichnet, dass** der Strom aus Siliciumdioxidvorläufer und Trägergas an einer oder mehreren Stellen im Reaktionsraum zugeführt wird.

7. Verfahren nach Anspruch 5 oder 6, **dadurch gekennzeichnet dass** das Mischoxidpulver gegebenenfalls durch eine Wärmebehandlung mittels Wasserdampf angefeuchteter Gase gereinigt wird.

8. Verfahren nach den Ansprüchen 5 bis 7, **dadurch gekennzeichnet, dass** als Titandioxidvorläufer und Siliciumdioxidvorläufer anorganische und/oder organische Verbindungen eingesetzt werden.

9. Verfahren nach den Ansprüchen 5 bis 8, **dadurch gekennzeichnet, dass** man als Titandioxidvorläufer Titantetrachlorid und als Siliciumdioxidvorläufer Siliciumtetrachlorid in einer Weise einleitet, dass sich das Gewichtsverhältnis SiO₂/TiO₂ auf der Oberfläche durch das Gewichtsverhältnis SiCl₄/TiCl₄ gemäß der Formel
[SiO₂/TiO₂] _{Oberfläche} = 7, 3 [SiCl₄/TiCl₄] ^{1,28},
mit einer absoluten Abweichung des Gewichtsverhältnisses [SiO₂/TiO₂] _{Oberfläche} von der angegebenen Formel von maximal 1,5 einstellt, mit der Maßgabe, daß das Gewichtsverhältnis SiO₂/TiO₂ im späteren Pulver beträgt zwischen 0,05 und 4, bezogen auf den Gesamtprimärpartikel.

10. Sonnenschutzmittel enthaltend das Pulver nach den Ansprüchen 1 bis 4, mit einem Anteil zwischen 0,01 und 25 Gew.-%, bezogen auf die Menge des Sonnenschutzmittels.

11. Verwendung des Mischoxidpulvers gemäß den Ansprüchen 1 bis 4 als UV-Filter, zur Herstellung von Dispersionen, in Prozessen zum chemisch-mechanischen Polieren (CMP-Anwendung), in der Glasherstellung, als katalytisch aktive Substanz, als Katalysatorträger, Verwendung in Tonern und als Additiv in der Silikon- und Kautschukindustrie.

## Claims

1. Silicon-titanium mixed oxide powder prepared by flame hydrolysis, **characterised in that** the ratio by weight of silicon dioxide / titanium dioxide on the surface of the primary particles is greater than that within the total primary particle.

2. Powder according to Claim 1, **characterised in that** the ratio by weight of SiO_{2/}TiO₂ is from 0.01 to 99, in relation to the total primary particle.

3. Powder according to Claim 1 or 2, **characterised in that** at a ratio by weight of SiO₂/TiO₂ of from 0.05 to 4, in relation to the total primary particle, the ratio by weight of SiO₂/TiO₂ on the surface assumes the value corresponding to the formula
[SiO₂/TiO₂] _{surface} = 9.3 [SiO₂/TiO₂] _{total primary particle}^{1.24},
wherein the maximum absolute deviation of the ratio by weight of [SiO₂/TiO₂] surface from the indicated formula is 1.5.

4. Powder according to Claims 1 to 3, **characterised in that** the BET surface area thereof is between 10 and 300 m²/g.

5. Process for the preparation of the powder according to Claims 1 to 4, **characterised in that** a stream consisting of a vaporous titanium dioxide precursor and oxygen or an oxygen-containing gas and hydrogen, and a second stream consisting of a vaporous silicon dioxide precursor and a carrier gas consisting of oxygen, an oxygen-containing gas and/or an inert gas, are guided separately into the reaction chamber of a burner such as is known for the preparation of pyrogenic oxides, and are burnt here, the solid mixed oxide powder and hot gases are subsequently cooled, and the gases are separated from the solid.

6. Process according to Claim 5, **characterised in that** the stream consisting of silicon dioxide precursor and carrier gas is supplied at one or more positions in the reaction chamber.

7. Process according to Claim 5 or 6, **characterised in that** the mixed oxide powder is optionally purified by a heat treatment by means of gases humidified with water vapour.

8. Process according to Claims 5 to 7, **characterised in that** inorganic and/or organic compounds are utilised as the titanium dioxide precursor and silicon dioxide precursor.

9. Process according to Claims 5 to 8, **characterised in that** titanium tetrachloride is introduced as the titanium dioxide precursor and silicon tetrachloride is introduced as the silicon dioxide precursor, in a manner such that the ratio by weight of SiO₂/TiO₂ on the surface becomes adjusted by the ratio by weight of SiCl₄/TiCl₄ corresponding to the formula
[SiO₂/TiO₂]_{surface} = 7.3 [SiCl₄/TiCl₄] ^{1.28},
with a 1.5 maximum absolute deviation of the ratio by weight of [SiO₂/TiO₂] _{surface} from the indicated formula, provided that the ratio by weight of SiO₂/TiO₂ in the subsequent powder is between 0.05 and 4, in relation to the total primary particle.

10. Sunscreen preparation containing the powder according to Claims 1 to 4, in a proportion of between 0.01 and 25 wt.%, in relation to the quantity of the sunscreen preparation.

11. Use of the mixed oxide powder according to Claims 1 to 4 as a UV filter, for the preparation of dispersions, in processes for chemical-mechanical polishing (CMP use), in glass manufacture, as a catalytically active substance, as a catalyst support, for use in toners and as an additive in the silicone and rubber industry.

## Revendications

1. Poudre d'oxyde mixte de titane et de silicium fabriquée par hydrolyse à la flamme,
**caractérisée en ce que**
le rapport pondéral entre le dioxyde de silicium et le dioxyde de titane à la surface des particules primaires, est plus important que dans la particule primaire totale.

2. Poudres selon la revendication 1,
**caractérisées en ce que**
le rapport pondéral SiO₂/TiO₂ est de 0,01 à 99, par rapport à la particule primaire totale.

3. Poudres selon la revendication 1 ou 2,
**caractérisées en ce que**
pour un rapport pondéral SiO_{2/}TiO₂ de 0,05 à 4, par rapport à la particule primaire totale, le rapport pondéral SiO_{2/}TiO₂ à la surface prend la valeur d'après la formule
**[SiO**_{**2**}**/TiO**_{**2**}**]** _{**surface**} = **9,3 [SiO**_{**2**}**/TiO**_{**2**}**]** _{**particule primaire totale**}^{**1,24**}
l'écart absolu entre le rapport pondéral [SiO₂/TiO₂]_{surface} et la formule indiquée étant au maximum de 1,5.

4. Poudres selon les revendications 1 à 3,
**caractérisées en ce que**
leur surface BET est comprise entre 10 et 300 m²/g.

5. Procédé de fabrication de la poudre selon les revendications 1 à 4,
**caractérisé en ce qu'**
on fait passer un courant constitué d'un précurseur, à l'état de vapeur, du dioxyde de titane et d'oxygène ou d'un gaz contenant de l'oxygène et d'hydrogène, et un deuxième courant constitué d'un précurseur, à l'état de vapeur, du dioxyde de silicium et d'un gaz support constitué d'oxygène, d'un gaz contenant de l'oxygène et/ou d'un gaz inerte, séparément dans la chambre de réaction d'un brûleur, comme celui que l'on connaît pour fabriquer des oxydes pyrogènes, chambre dans laquelle le mélange est brûlé, on fait ensuite refroidir la poudre d'oxyde mixte solide et les gaz très chauds et on sépare les gaz de la substance solide.

6. Procédé selon la revendication 5,
**caractérisé en ce qu'**
on fait passer le courant constitué de précurseur du dioxyde de silicium et de gaz support à un ou plusieurs endroits à l'intérieur de la chambre de réaction.

7. Procédé selon la revendication 5 ou 6,
**caractérisé en ce qu'**
on purifie la poudre d'oxyde mixte le cas échéant par un traitement thermique des gaz humidifiés avec de la vapeur d'eau.

8. Procédé selon les revendications 5 à 7,
**caractérisé en ce que**
comme précurseur du dioxyde de titane et précurseur du dioxyde de silicium, on utilise des composés inorganiques et/ou organiques.

9. Procédé selon les revendications 5 à 8,
**caractérisé en ce qu'**
on introduit du tétrachlorure de titane comme précurseur du dioxyde de titane, et du tétrachlorure de silicium comme précurseur du dioxyde de silicium, de manière à ce que le rapport pondéral SiO₂/TiO₂ à la surface puisse être ajusté par le rapport pondéral SiCl_{4/}TiCl₄, selon la formule
**[SiO**_{**2**}**/TiO**_{**2**}**]** _{**surface**} = **7,3 [SiCl**_{**4**}**/TiCl**_{**4**}**]**^{**1,28**}
avec un écart absolu du rapport pondéral [SiO₂/TiO₂]_{surface} par rapport à la formule indiquée d'au maximum 1,5, à condition que le rapport pondéral SiO₂/TiO₂ soit compris ensuite, dans la poudre, entre 0,05 et 4, par rapport à la particule primaire totale.

10. Produit antisolaire contenant la poudre selon les revendications 1 à 4, dans une proportion comprise entre 0,01 et 25 % en poids par rapport à la quantité totale de produit antisolaire.

11. Utilisation de la poudre d'oxyde mixte selon les revendications 1 à 4, en tant que filtres UV, pour préparer des dispersions, dans des processus pour le polissage chimio-mécanique (application CMP), dans la fabrication du verre, en tant que substance à action catalytique, en tant que support de catalyseur, utilisation dans des toners et en tant qu'additif dans l'industrie du silicone et du caoutchouc.
